# EUROPÄISCHE PATENTSCHRIFT

(11) **EP 1 990 154 B1**
(45) Veröffentlichungstag und Bekanntmachung des Hinweises auf die Patenterteilung: **12.11.2014**
(21) Anmeldenummer: 08103280.7
(22) Anmeldetag: 31.03.2008
(51) Int. Cl.: B27G 15/00, B23B 51/00

(54) **Schneidelement für ein Schneidwerkzeug**
Cutting element for a cutting tool
Elément de coupe pour un outil de coupe

(30) Priorität: 11.05.2007 AT 7372007
(43) Veröffentlichungstag der Anmeldung: 12.11.2008
(73) Patentinhaber: BOEHLERIT GmbH & Co.KG., 8605 Kapfenberg (AT); Leitz GmbH & Co. KG, 73447 Oberkochen (DE)
(72) Erfinder: Kisselbach, Andreas, 73431, Aalen (DE); Hammer, Helmut, 73431, Aalen (DE); Bärnthaler, Walter, 8641, St. Marein im Mürztal (AT); Feistritzer, Stefan, 8020, Graz (AT)
(74) Vertreter: Wirnsberger, Gernot

(56) Entgegenhaltungen:
- EP-A2- 1 319 483
- WO-A1-89/00097
- DE-A1- 2 636 182
- DE-A1- 19 832 551
- DE-C- 331 207
- DE-C1- 3 523 452
- DE-U1- 9 417 778
- GB-A- 328 153
- US-A- 458 640
- US-A- 877 831
- US-A- 1 444 626
- US-A- 2 883 888
- US-A1- 2005 249 563
- US-B1- 6 354 774

## Beschreibung

Die Erfindung betrifft ein Schneidelement aus einem Hartmetall für ein Schneidwerkzeug wie einen Bohrer oder Fräser, mit einem etwa zylinderförmigen Mittelteil, auf dessen einem Ende eine Schneidspitze und auf dessen anderem Ende eine Lötspitze sitzt, sowie mit sich radial vom Mittelteil nach außen erstreckenden, vorzugsweise rotationssymmetrisch zur Achse des Mittelteiles ausgebildeten Flügeln, die jeweils eine der Schneidspitze zugewandte Stirnseite mit einer außenseitig angeordneten Vorschneide und einer vom Mittelteil nach außen verlaufenden Hauptschneide sowie eine gegenüberliegende, der Lötspitze zugewandte Befestigungsseite aufweisen, wobei die Flügel an der Stirnseite mit einem an die Hauptschneide anschließenden abfallenden Rücken gemäß dem Oberbegriff des Anspruchs 1 ausgebildet sind.

Ein solches Schneidelement ist aus der EP 1 319 483 bekannt.

Des Weiteren betrifft die Erfindung ein Schneidwerkzeug mit einem derartigen Schneidelement.

Schneidelemente der eingangs genannten Art werden beispielsweise bei Schaftfräsern oder Beschlagbohrern eingesetzt. Solche Schneidelemente werden gemäß dem Stand der Technik bevorzugt durch Powder-Injection-Molding (PIM) bzw. Pulverspritzgießen gefertigt und anschließend durch Schleifen in einen einsatzfähigen Gebrauchszustand gebracht. Mit zunehmender Gebrauchs- bzw. Einsatzdauer ist es sodann auch erforderlich, die Hauptschneiden nachzuschärfen.

Wenngleich die Schneidelemente im PIM-Verfahren insbesondere auch aus verschleißfesten Hartmetallen erstellt werden können, so weist dieses Verfahren dennoch die Nachteile auf, dass es einerseits teuer ist und andererseits oftmals bei der Herstellung, insbesondere im Rahmen der notwendigen Behandlung der Grünlinge, ein Verzug der Schneidelementrohlinge auftritt. Bei bekannten Schneidelementen ist auch nicht berücksichtigt, dass im Gebrauch durch das regelmäßig erforderliche Nachschleifen bzw. Schärfen der Hauptschneide, welches zwangsweise einen Abtrag von Hartmetall vom Schneidelement verursacht, eine nicht kompensierbare Schwächung desselben gegeben ist, mit dem nach Nachteil, dass dieses nach wenigen Nachschleifoperationen nicht mehr höchsten Schneidbelastungen standhalten kann.

Hier setzt die Erfindung an und stellt sich die Aufgabe, ein Schneidelement der eingangs genannten Art anzugeben, welches im Wesentlichen verzugsfrei herstellbar und mehrmals nachschleifbar ist, ohne seine Stabilität zu verlieren.

Ein weiteres Ziel der Erfindung besteht darin, ein Schneidwerkzeug der eingangs genannten Art anzugeben, dessen Schneidelement mehrmals nachschleifbar ist, ohne dass bei Gebrauch des Schneidwerkzeuges ein Bruch des Schneidelementes befürchtet werden müsste.

Das erste Ziel der Erfindung wird dadurch erreicht, dass bei einem Schneidelement der eingangs genannten Art die Flügel auf der Befestigungsseite in einem dem Rücken entsprechenden Bereich mit etwa gleicher Kontur verstärkt ausgebildet sind, sodass die Flügel im Querschnitt zur Hauptschneide betrachtet ungefähr die gleiche Stärke aufweise.

Wie von den Erfindern bereits in Erwägung gezogen wurde, sollten sich verzugsfreie Schneidelemente herstellen lassen, wenn anstelle eines PIM-Verfahrens ein herkömmliches Pressen von Hartmetallen, gefolgt von einem Sintern, angewandt wird. Dabei tritt nach Erkenntnis der Erfinder allerdings das Problem auf, dass bei den bekannten geometrischen Strukturen von Schneidelementen - mit im Querschnitt stark variierender Stärke der Flügel - ein Pressen stets zu einer äußerst inhomogenen Materialverteilung führt, sodass die erstellten Schneidelemente letztlich viele Schwachstellen aufweisen, beispielsweise in jenen Bereichen, die querschnittlich betrachtet dünner ausgeformt sind.

Wird nun, wie von den Erfindern erkannt und erfindungsgemäß vorgesehen, die Befestigungsseite in einem dem Rücken entsprechenden Bereich mit etwa gleicher Kontur verstärkt ausgebildet, so weisen die Flügel im Querschnitt zur Hauptschneide betrachtet ungefähr gleiche Stärke auf, wodurch bei einem Pressen des Hartmetalls eine Ausbildung von Inhomogenitäten vermieden wird. Mit anderen Worten: Durch eine ausgewogene Materialverteilung und gleichmäßig einwirkende Presskräfte kommt es in Bezug auf ein Gefüge im Wesentlichen zu einer vollständig homogenen Ausbildung der Flügel im gesamten Bereich. Inhomogenitäten wie Poren oder unerwünschte Dichteunterschiede und sich daraus ergebender starker Verzug beim Sintern können dadurch vermieden werden.

Durch die verstärkte Ausbildung im Bereich der Befestigungsseite ist zwar mehr Hartmetall vorzusehen, allerdings wird dadurch auch erreicht, dass das Schneidelement an der Stirnseite mehrmals nachschleifbar ist, ohne dass es bereichsweise so dünn wird, dass im Schneideinsatz eine Bruchgefahr gegeben wäre.

Da das Schneidelement möglichst oft nachschleifbar sein soll, ist es zweckmäßig, dass die Flügel im Bereich der Hauptschneide eine größte Dicke bzw. Stärke aufweisen, sodass sich ein mit einem Schleifen bzw. Schärfen einhergehender Materialabtrag möglichst wenig auswirkt. Eine Dicke bzw. Stärke, wie sie im Bereich der Hauptschneide gegeben ist, soll jedoch quer zur Hauptscheide betrachtet um nicht mehr als 40 %, vorzugsweise nicht mehr als 25 %, abfallen, um eine homogene Gefügeausbildung beim Pressen nicht zu gefährden.

Diesbezüglich hat es sich besonders bewährt, dass der Rücken von der Hauptschneide bis zur gegenüberliegenden Seite des Flügels abfallend verläuft und mit einem ebenen Bereich sowie einem an den ebenen Bereich anschließenden, stärker abfallenden, vorzugsweise konkav gekrümmten Bereich, der in einen zweiten ebenen, zum ersten ebenen Bereich etwa parallel verlaufenden Bereich übergeht, ausgebildet ist. Da der erste ebene Bereich, zusammen mit einer Seitenfläche, eine Hauptschneide bestimmt, die durch Schärfen bzw. regelmäßiges Nachschärfen funktionstüchtig gemacht wird, ist für den Benutzer eine Art Gebrauchsindikator gegeben: Solang bei einem Nachschärfen der konkave Bereich zumindest teilweise erhalten bleibt, weist das Schneidelement eine erforderliche Stärke auf, die es für einen Schneideinsatz brauchbar macht. Sobald durch oftmaliges Nachschärfen auch der konkave Bereich abgeschliffen ist und der erste ebene Bereich in den zweiten ebenen Bereich übergeht bzw. mit diesem eine Ebene bildet, wird dem Benutzer signalisiert, dass das Schneidelement nunmehr eine minimale Dicke aufweist und gerade noch für die Dauer einer geeigneten Schärfe der Hauptschneide einsetzbar ist. Ein weiteres Nachschärfen würde das Schneidelement derart schwächen, dass bei weiterem Gebrauch des Schneidelementes ein Bruch desselben mit hoher Wahrscheinlichkeit möglich ist.

In Bezug auf eine möglichst lange Gebrauchsdauer des Schneidelementes kann auch vorgesehen sein, dass sich die Vorschneide zumindest über 75 % eines äußeren Umfangs des Flügels erstreckt.

Ebenfalls in Bezug auf eine möglichst lange Gebrauchsdauer des Schneidelementes ist vorgesehen, dass die Vorschneide bogenförmig ausgebildet ist. Die Hauptschneiden hingegen sind vorzugsweise gerade ausgebildet.

Entsprechend den geschilderten Vorteilen der Erfindung erweist es sich als zweckmäßig, dass das Schneidelement einteilig aus einem gepressten und gesinterten Hartmetall gebildet ist.

Das weitere Ziel der Erfindung wird im Sinne der vorstehend erläuterten Vorteile dadurch erreicht, dass ein Schneidwerkzeug mit einem Schaft und einem Schneidelement wie dargelegt ausgebildet ist, wobei das Schneidelement am Schaft angelötet oder integral mit diesem erstellt ist.

Weitere Merkmale, Vorteile und Wirkungen der Erfindung ergeben sich aus dem Zusammenhang der Beschreibung und den nachfolgenden Ausführungsbeispielen, anhand derer die Erfindung noch weitergehend erläutert ist.

Es zeigen:
Fig. 1 ein erfindungsgemäßes Schneidelement in Draufsicht;
Fig. 2 ein erfindungsgemäßes Schneidelement im Querschnitt entlang der Linie II-II in Fig. 1;
Fig. 3 einen Querschnitt (nicht maßstabsgetreu) entlang der Linie III-III in Fig. 1;
Fig. 4 einen Querschnitt (nicht maßstabsgetreu) entlang der Linie IV-IV in Fig. 1;
Fig. 5 ein erfindungsgemäßes Schneidwerkzeug.

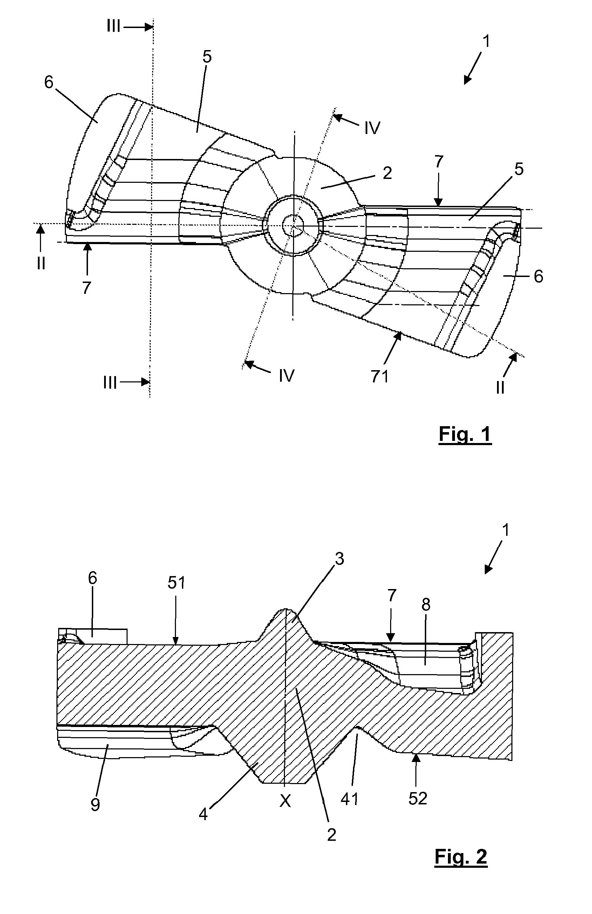

In Fig. 1 ist ein erfindungsgemäßes Schneidelement 1 in Draufsicht näher dargestellt. Das Schneidelement 1 weist einen zentralen, in Draufsicht im Wesentlichen runden Mittelteil 2 auf, an dem zwei in einem Winkel von 180° befindliche Flügel integral angeformt sind. Das Schneidelement 1 besteht zur Gänze aus Hartmetall und ist durch Pressen und Sintern pulverförmiger Ausgangskomponenten erstellt.

Der Mittelteil 2 weist zwei Enden auf, wobei zentrisch auf einer Achse X an einem Ende des Mittelteiles 2 eine Schneidspitze 3 und an einem gegenüberliegenden Ende eine Lötspitze 4 positioniert sind (Fig. 2). An dem Mittelteil 2 sind zwei Flügel 5 wie erwähnt integral angeformt, die an einer Stirnseite 51, das ist jene Seite, die der Schneidspitze 3 zugewandt ist, eine sogenannte Vorschneide 6 und eine Hauptschneide 7 aufweisen. Im Schneideinsatz sorgt die Vorschneide 6 dafür, beispielsweise bei einer spanenden Bearbeitung von Holzwerkstoffen, dass Fasern durchtrennt werden. Die Räum- bzw. Hauptschneide 7 bewirkt eine Spanabnahme. Dementsprechend ist, wie anhand der Fig. 2 ersichtlich, die Vorschneide 6 höher positioniert als die Hauptschneide 7. Die noch weiter vorragende Bohr- bzw. Schneidspitze 3 sorgt dafür, dass das Schneidwerkzeug 10 stets zentriert bleibt.

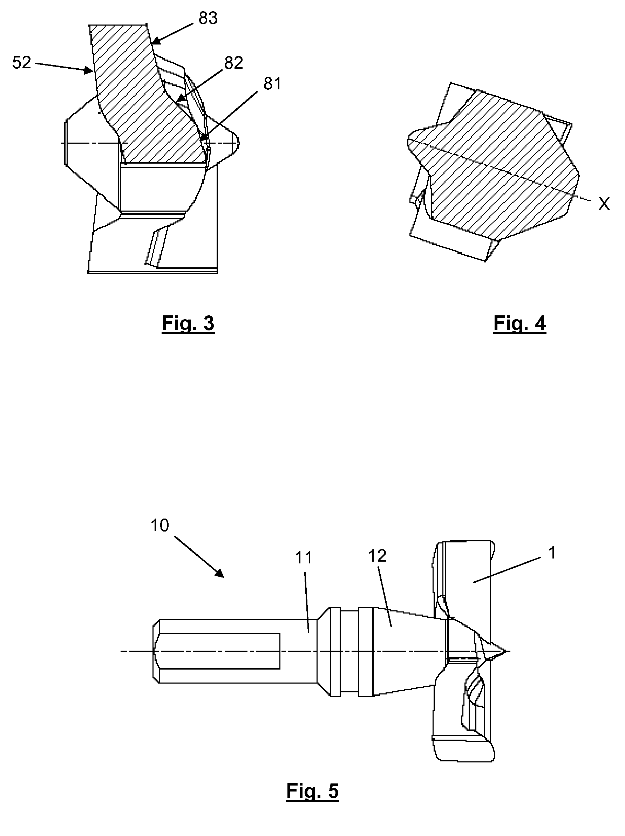

Wie aus Fig. 1 ersichtlich, sind die Flügel 5 in Draufsicht mit einer geraden Hauptschneide 7 ausgebildet, an die im außenseitigen Bereich eine Vorschneide 6 anschließt. Die Vorschneide 6 ist möglichst nahe an die Hauptschneide 7 gesetzt und erstreckt sich vorzugsweise zumindest über 75 % eines äußeren, bogenförmig ausgebildeten Umfangs eines Flügels 5. Diese bogenförmige Vorschneide 6 geht ihrerseits in eine der Hauptschneide 7 gegenüberliegenden, in Draufsicht ebenfalls gerade ausgebildete Kante 71 bzw. Seitenfläche über. Zwischen der Kante 71 und der Hauptschneide 7 ist ein Rücken 8 ausgebildet, der von der Hauptschneide 7 bis zur gegenüberliegenden Kante 71 abfällt, wie insbesondere anhand der Fig. 3 ersichtlich. Dabei ist der Rücken 8 in einzelne Bereiche 81, 82 und 83 unterteilt, denen an einer Rückseite bzw. Befestigungsseite 52 eine der Kontur des Rückens 8 ungefähr nachgebildete Verstärkungszone 9 entspricht. Durch diese Verstärkungszone 9 wird erreicht, dass das Schneidelement 1 im Querschnitt betrachtet lediglich geringe Variationen in seiner Stärke aufweist, weshalb es mittels Pressens im Wesentlichen mit homogener Gefügeausbildung erstellt werden kann. Die aus Fig. 3 ersichtlichen Stärkeunterschiede sind darauf zurückzuführen, dass das Schneidelement 1 im Bereich einer Hauptschneide 7 oftmals nachschleifbar bzw. schärfbar sein soll, weshalb in diesem Bereich quasi ein gewisser Materialüberschuss bzw. eine stärkere Ausbildung vorgesehen ist. Dabei sind, wie in Fig. 3 dargestellt, die Bereiche 81 und 83 im Wesentlichen parallel zueinander verlaufend ausgebildet, wohingegen der am stärksten abfallenden Bereich 82 teilweise eine konkav gekrümmte Struktur aufweist. Dadurch wird einerseits erreicht, dass, zumindest bei geringer Gebrauchsdauer, nur eine kleine Fläche, nämlich im Wesentlichen der Bereich 81, nachzuschärfen ist. Andererseits wird bei mehrmaligem Nachschleifen der konkave Bereich 82 langsam abgenommen, bis letztlich der Bereich 81 mit dem Bereich 83 parallel verlaufend ausgebildet ist bzw. eine Ebene bildet. Diese Situation zeigt dem Benutzer bzw. Werkzeuggebraucher an, dass das Schneidelement 1 nunmehr eine im Querschnitt betrachtet im Wesentlichen gleiche Stärke aufweist und daher gerade noch einmal eingesetzt werden kann, ehe ein Versagen des Schneidelementes 1 im Gebrauch sehr wahrscheinlich wird.

Zwischen den Flügeln 5 und der Lötspitze 4 können entsprechende Freistellungen 41 vorgesehen sein, an welchen ein einseitiger Teil 12 eines Schaftes 11 eines Schneidwerkzeuges 10 zur Anlage kommt, wenn das Schneidelement 1 mit dem Schaft 11 durch Löten verbunden werden soll (Fig. 5). Dadurch ist, ohne das Schneidelement 1 in seiner Stabilität zu beeinträchtigen, eine sichere Verbindung des Schaftes 11 mit dem Schneidelement 1 durch Löten möglich.

Die Herstellung eines erfindungsgemäßen Schneidelementes 1 kann erfolgen, indem ein Hartmetall in Pulverform in eine Matrize eingebracht und mit einem Pressdruck von ca. 1 bis 2 T/cm² zu einem Formteil gepresst wird. Anschließend wird das so erstellte Formteil bei ca. 1200 °C bis 1400 °C beispielsweise im Vakuum gesintert. Nach dem Sintern und Abkühlen auf Umgebungstemperatur werden die Hauptschneiden 7 geschärft und das Schneidelement 1 ist einsatzbereit.

## Patentansprüche

1. Schneidelement (1) aus einem Hartmetall für ein Schneidwerkzeug (10) wie einen Bohrer oder Fräser, mit einem etwa zylinderförmigen Mittelteil (2), auf dessen einem Ende eine Schneidspitze (3) und auf dessen anderem Ende eine Lötspitze (4) sitzt, sowie mit sich radial vom Mittelteil (2) nach außen erstreckenden, vorzugsweise rotationssymmetrisch zur Achse (X) des Mittelteiles (2) ausgebildeten Flügeln (5), die jeweils eine der Schneidspitze (3) zugewandte Stirnseite (51) mit einer außenseitig angeordneten Vorschneide (6) und einer vom Mittelteil (2) nach außen verlaufenden Hauptschneide (7) sowie eine gegenüberliegende, der Lötspitze (4) zugewandte Befestigungsseite (52) aufweisen, wobei die Flügel (5) an der Stirnseite (51) mit einem an die Hauptschneide (7) anschließenden abfallenden Rücken (8) ausgebildet sind, **dadurch gekennzeichnet, dass** die Flügel (5) auf der Befestigungsseite (52) in einem dem Rücken (8) entsprechenden Bereich (9) mit etwa gleicher Kontur verstärkt ausgebildet sind sodass die Flügel (5) im Querschnitt zur Hauptschneide (7) betrachtet ungefähr gleiche Stärke aufweisen.

2. Schneidelement (1) nach Anspruch 1, **dadurch gekennzeichnet, dass** die Flügel (5) im Bereich der Hauptschneide (7) eine größte Dicke aufweisen.

3. Schneidelement (1) nach Anspruch 2, **dadurch gekennzeichnet, dass** der Rücken (8) von der Hauptschneide (7) bis zur gegenüberliegenden Seite des Flügels (5) abfallend verläuft und mit einem ebenen Bereich (81) sowie einem an den ebenen Bereich (81) anschließenden, stärker abfallenden, vorzugsweise konkav gekrümmten Bereich (82), der in einen zweiten ebenen, zum ersten ebenen Bereich (81) etwa parallel verlaufenden Bereich (83) übergeht, ausgebildet ist.

4. Schneidelement (1) nach einem der Ansprüche 1 bis 3, **dadurch gekennzeichnet, dass** sich die Vorschneide (6) zumindest über 75 % eines äußeren Umfangs des Flügels (5) erstreckt.

5. Schneidelement (1) nach einem der Ansprüche 1 bis 4, **dadurch gekennzeichnet, dass** die Vorschneide (6) bogenförmig ausgebildet ist.

6. Schneidelement (1) nach einem der Ansprüche 1 bis 5, **dadurch gekennzeichnet, dass** die Hauptschneide (7) gerade ausgebildet ist.

7. Schneidelement (1) nach einem der Ansprüche 1 bis 6, **dadurch gekennzeichnet, dass** das Schneidelement (1) einteilig aus einem gepressten und gesinterten Hartmetall gebildet ist.

8. Schneidwerkzeug (10) mit einem Schaft (11) und einem Schneidelement (1) nach einem der Ansprüche 1 bis 7, welches am Schaft (11) angelötet oder integral mit diesem erstellt ist.

## Claims

1. A cutting element (1) from a carbide for a cutting tool (10) such as a drill or milling cutter, having an approximately cylindrical central part (2) on the one end of which a cutting tip (3) is located and on the other end of which a soldering tip (4) is located, and having wings (5) which extend radially outwards from the central part (2) and are preferably formed to be rotationally symmetric to the axis (X) of the central part (2), each of the wings having a front side (51) that faces towards the cutting tip (3) and that has a rough-cutting edge (6) arranged on the outside and a main cutting edge (7) extending outwards from the central part (2), and having an opposing attachment side (52) that faces towards the soldering tip (4), wherein the wings (5) are formed on the front side (51) with a sloping ridge (8) connected to the main cutting edge (7), **characterized in that** on the attachment side (52), the wings (5) are formed to be reinforced in a region which (9) corresponds to the ridge (8) and which has approximately the same contour so that the wings (5), viewed in cross-section towards the main cutting edge (7), have approximately the same thickness.

2. The cutting element (1) according to claim 1, **characterized in that** the wings (5) have a maximum thickness in the region of the main cutting edge (7).

3. The cutting element (1) according to claim 2, **characterized in that** the ridge (8) is sloping from the main cutting edge (7) up to the opposing side of the wing (5) and is formed with a flat region (81) and with a more steeply sloping region (82) which is connected to the flat region (81) and which is preferably concavely curved and which transitions into a second flat region (83) that runs approximately parallel to the first flat region (81).

4. The cutting element (1) according to any one of the claims 1 to 3, **characterized in that** the rough-cutting edge (6) extends at least over 75% of an outer circumference of the wing (5).

5. The cutting element (1) according to any one of the claims 1 to 4, **characterized in that** the rough-cutting edge (6) is arc-shaped.

6. The cutting element (1) according to any one of the claims 1 to 5, **characterized in that** the main cutting edge (7) is formed straight.

7. The cutting element (1) according to any one of the claims 1 to 6, **characterized in that** the cutting element (1) is formed as one piece from a pressed and sintered carbide.

8. A cutting tool (10) having a shank (11) and a cutting element (1) according to any one of the claims 1 to 7, which is soldered to the shank (11) or is formed integrally with said shank.

## Revendications

1. Elément de coupe (1) en métal dur pour un outil de coupe (10) comme une foret ou une fraise avec une partie centrale (2) cylindrique sur une extrémité duquel est située une pointe coupante (3) et sur l'autre extrémité une pointe à souder (4) ainsi qu'avec des ailettes (5) constituées en s'étendant radialement de la partie centrale (2) vers l'extérieur, de préférence de façon symétrique en rotation par rapport à l'axe (X) de la partie centrale (2) qui comportent respectivement une face avant (51) tournée vers la pointe coupante (3) avec un arête de coupe d'ébauche (6) disposé extérieurement et une arête de coupe principale (7) passant vers l'extérieur de la partie centrale (2) ainsi qu'un côté de fixation (52) opposé tourné vers la pointe à souder (4), pour lequel les ailettes (5) sont constituées sur la face avant (51) avec un arrière (8)incliné se raccordant à l'arête de coupe principale (7), **caractérisé en ce que** les ailettes (5) sont constituées sur le côté de fixation (52) renforcées dans une zone (9) correspondant à l'arrière (8) avec à peu près le même profil de sorte que les ailettes (5) présentent à peu près la même épaisseur vues en coupe transversale de l'arête de coupe principale (7).

2. Elément de coupe (1) selon la revendication 1, **caractérisé en ce que** les ailettes (5) présentent une épaisseur maximale dans la zone de l'arête de coupe principale (7).

3. Elément de coupe (1) selon la revendication 2, **caractérisé en ce que** l'arrière (8) est incliné de l'arête de coupe principale (7) jusqu'au côté opposé de l'ailette (5) et est constitué avec une zone (81) plane ainsi qu'une zone (82)plus fortement inclinée, se raccordant à la zone (81) plane, de préférence courbée de façon concave qui passe dans une deuxième zone (83) plane passant à peu près parallèlement à la première zone (81) plane.

4. Elément de coupe (1) selon une quelconque des revendications 1 à 3, **caractérisé en ce que** l'arête de coupe d'ébauche (6) s'étend au moins sur 75% d'une périphérie extérieure de l'ailette (5).

5. Elément de coupe (1) selon une quelconque des revendications 1 à 4, **caractérisé en ce que** l'arête de coupe d'ébauche (6) est constituée en forme d'arc.

6. Elément de coupe (1) selon une quelconque des revendications 1 à 5, **caractérisé en ce que** l'arête de coupe principale (7) est constituée droite.

7. Elément de coupe (1) selon une quelconque des revendications 1 à 6, **caractérisé en ce que** l'élément de coupe (1) est formé d'une seule pièce en métal dur matricé et fritté.

8. Outil de coupe (10) avec une queue (11) et un élément de coupe (1) selon une quelconque des revendications 1 à 7 qui est soudé à la queue (11) ou réalisé de façon intégrale avec celle-ci.
